# FASCICULE DE BREVET EUROPEEN

(11) **EP 0 566 877 B1**
(45) Date de publication et mention de la délivrance du brevet: **27.11.2002**
(21) Numéro de dépôt: 93104638.7
(22) Date de dépôt: 22.03.1993
(51) Int. Cl.: C12P 21/06, C12M 1/06, A23J 3/34

(54) **Hydrolyse enzymatique de protéines**
Enzymatische Hydrolyse von Proteinen
Enzymatic hydrolysis of proteins

(30) Priorité: 09.04.1992 CH 116092
(43) Date de publication de la demande: 27.10.1993
(73) Titulaire: SOCIETE DES PRODUITS NESTLE S.A., 1800 Vevey (CH)
(72) Inventeur: Baensch, Johannes, CH-1009 Pully (CH); Margot, Antoine, CH-1015 Lausanne (CH); Meister, Niklaus, CH-3506 Grosshoechstetten (CH); Renken, Albert, CH-1015 Lausanne (CH); Wood, Robert Dustan, CH-1004 Lausanne (CH); Woupeyi, Alfred, CH-1400 Yverdon (CH)
(74) Mandataire: Wavre, Claude-Alain

(56) Documents cités:
- EP-A- 0 274 946
- EP-A- 0 286 838
- US-A- 3 442 656
- US-A- 4 464 509
- US-A- 5 174 651
- DATABASE WPI Week 8815, Derwent Publications Ltd., London, GB; AN 88-099079 & DD-A-251 539 (VEB LEUNA-WERK ULBRIGHT) 18 Novembre 1987

## Description

La présente invention a pour objet un procédé d'hydrolyse enzymatique de protéines et un appareil pour la mise en oeuvre du procédé.

On connaît divers procédés d'hydrolyse enzymatique de protéines qui se distinguent par le choix du substrat, de l'enzyme, du degré d'hydrolyse et/ou du profil peptidique recherché, par exemple. Lorsque, pour des raisons d'assimilation de l'hydrolysat par la muqueuse intestinale, par exemple, un profil peptidique relativement bien défini, notamment un profil oligopeptidique étroit est recherché, les procédés d'hydrolyse connus comportent généralement au moins une étape de filtration ou tamisage de l'hydrolysat.

C'est ainsi que EP 226221, par exemple, décrit un procédé de préparation de peptides hypoallergéniques de poids moléculaire compris entre 2000 et 6000 par une ou plusieurs étapes d'hydrolyse enzymatique de protéines réalisées chacune en discontinu en cuve de fermentation et terminées chacune par une étape d'ultrafiltration.

US 4212889 décrit un procédé de solubilisation de protéines de poisson, dans lequel on fait passer en continu un mélange de chair de poisson et d'enzyme au travers d'une installation comprenant plusieurs cuves d'hydrolyse branchées en série.

EP-A-0322589 décrit un procédé de préparation d'un hydrolysat de protéine de lactosérum et d'un aliment hypoallergénique, dans lequel on hydrolyse enzymatiquement un produit lactosérique en deux étapes d'hydrolyse distinctes, l'hydrolysat de la première hydrolyse étant traité thermiquement de manière à dénaturer les protéines restées intactes après la première hydrolyse et à les rendre ainsi susceptibles à la seconde hydrolyse, de sorte qu'après la seconde hydrolyse l'hydrolysat soit exempt d'allergènes tels que protéines, fragments de protéines ou macropeptides de poids moléculaire supérieur à 10'000.

La présente invention a pour but de proposer un procédé d'hydrolyse qui, tout en étant réalisé en continu, permette d'égaler, voire d'améliorer l'efficacité d'une hydrolyse en discontinu en cuve et qui permette d'obtenir un hydrolysat protéique présentant un degré d'hydrolyse et/ou un spectre peptidique bien défini et reproductible.

A cet effet, dans le procédé d'hydrolyse enzymatique de protéines selon la présente invention, on soumet un substrat protéique à une hydrolyse en continu avec une enzyme protéolytique, on réalise une première étape d'hydrolyse enzymatique relativement courte en cuve sous agitation et une deuxième étape d'hydrolyse enzymatique relativement longue en tube, on réalise la première étape durant 10-60 min tout en ajustant le pH et la température à des valeurs favorables à l'activité de l'enzyme, on réalise la seconde étape durant 1-8 h en ajustant la température à une valeur égale ou supérieure à la température de la première étape, et l'on réalise la deuxième étape d'hydrolyse en tube garni d'éléments mélangeurs statiques.

De même, l'appareil pour la mise en oeuvre du présent procédé comprend une cuve d'hydrolyse à double manteau avec agitateur reliée en amont à un dispositif doseur de substrat et à un dispositif doseur d'enzyme, et reliée en aval à au moins un tube d'hydrolyse, ledit tube étant garni d'éléments mélangeurs statiques et étant de relativement grande dimension, et la cuve étant de relativement faible dimension.

On a constaté qu'il était possible ainsi de produire, de préférence en continu, avec une bonne efficacité et une bonne reproductibilité, un hydrolysat protéique présentant un degré d'hydrolyse et/ou un spectre peptidique bien définis.

Grâce à ce procédé et à cet appareil, il est en particulier possible de travailler avec une cuve de relativement faible dimension que l'on peut remplir complètement, sans laisser subsister d'espace de tête, et avec un tube de relativement grande dimension. On peut donc réaliser en continu, une première étape relativement courte, en d'autres termes une étape de lancement de l'hydrolyse dans une relativement petite cuve, et une deuxième étape relativement longue, en d'autres termes une étape de finition de l'hydrolyse dans un tube présentant un volume relativement grand. Il est ainsi possible de contrôler de manière précise et simple, par exemple par l'intermédiaire de pompes volumétriques, le temps de réaction, autrement dit le temps de résidence du substrat dans le volume total représenté par la somme du volume de la cuve et du volume du tube.

Si, pour comparaison, on désire réaliser une hydrolyse dans une seule grande cuve d'hydrolyse, le temps de résidence d'un volume élémentaire d'hydrolysat ne peut pas être défini de manière précise. Ceci est vrai pour une hydrolyse en discontinu, les temps nécessaires pour établir des conditions données de pH et/ou de température, voire pour vider la cuve, par exemple, n'étant pas négligeables. Mais ceci est d'autant plus vrai pour une hydrolyse en continu, pour laquelle il n'est possible de définir qu'un temps de résidence moyen. Même dans un procédé tel que celui d'US 4212889 cité plus haut, le temps de résidence ne peut guère être défini de manière plus précise.

Par contre, il s'avère qu'avec le procédé et l'appareil selon la présente invention, le temps de résidence d'un volume élémentaire d'hydrolysat peut être défini de manière remarquablement précise, le courant de l'hydrolysat dans le tube d'hydrolyse, de préférence garni d'éléments mélangeurs statiques, pouvant présenter un front très plat.

Dans le présent exposé, on définit un degré d'hydrolyse par l'intermédiaire de la quantité d'azote non protéique (NPN) déterminée comme le pourcentage de l'azote total non précipitable à l'acide trichloracétique à 13%.

Les teneurs en azote sont déterminées par la méthode Kjeldahl.

Les teneurs en azote aminé (alpha-NH₂ libre) sont déterminées par réaction à la ninhydrine après hydrolyse alcaline.

Les tests de relâchement de sérotonine exogène marquée au tritium (sérotonine-³H) sont faits sur des mastocytes normaux de la cavité péritonéale du rat selon la méthode décrite par R.Fritsché et M.Bonzon dans Int Arch Allerg Immunol 93, 289-293 (1990).

Les tests d'inhibition ELISA sont faits avec des anticorps de lapin spécifiques de la beta-lactoglobuline (BLG), de la sérumalbumine bovine (BSA) et de la caséine (CAS). La sensibilité de la méthode, autrement dit la limite de concentration de détection est de 20 ng/ml.

Les analyses par chromatographie liquide haute performance (tests HPLC, profils peptidiques) sont effectuées dans des conditions non dénaturantes sur gel à base de silice type TSK-G2000-SW de la maison Toyo Soda, dont le domaine de fractionnement s'étend de 500 à 50000 Dalton, dans une colonne BIOSIL SEC-125 de la maison BIORAD. Les résultats sont exprimés en % de distribution de surface des pics lus à 220 nm en solution phosphate 0,1 M + NaCl 0,4 M à pH 6,80 .

Les analyses par électrophorèse de zone en gel de polyacrylamide (tests SDS-PAGE) sont effectuées selon la méthode décrite par Laemmli dans Nature 227, 680 et suivantes (1970).

Le blocage de lysine est déterminé par HPLC et exprimé en % de lysine bloquée par rapport à la lysine totale de l'hydrolysat.

Par l'expression "éléments mélangeurs statiques", on peut comprendre des croisillons ou des lamelles ondulées métalliques ou en plastique qui se croisent ou sont imbriqués les uns dans les autres et qui subdivisent l'espace délimité par ledit tube en une pluralité de canaux qui se croisent tout en progressant dans l'axe du tube. De tels éléments sont commercialisés par la maison Sulzer A.G., CH-8401 Winterthur, sous la désignation SMV, SMX ou SMXL, par exemple.

Enfin, dans le présent exposé, il faut comprendre que lesdits tubes garnis d'éléments mélangeurs statiques sont systématiquement munis d'un double manteau, même quand ça n'est pas précisé.

Pour mettre en oeuvre le présent procédé, on peut utiliser comme substrat protéique toute matière première alimentaire riche en protéines, telle que des farines ou semoules de graines ou de tourteaux d'oléagineuses, des levures ou bactéries alimentaires, de la chair animale ou de poisson hachée, ou des laits ou dérivés du lait, sous forme de particules en suspension aqueuse ou de suspension aqueuse, par exemple.

De préférence, ledit substrat protéique est un substrat lactosérique contenant les protéines du lactosérum, notamment un lactosérum doux de fromagerie ou un lactosérum acide de caséinerie, tel quel ou sous forme déminéralisée ou délactosée, liquide ou reconstitué.

De préférence également, on choisit l'enzyme dans le groupe formé par la trypsine, la chymotrypsine, la pancréatine, les protéases bactériennes, les protéases fongiques, et leurs mélanges.

On peut mélanger l'enzyme protéolytique et le substrat à raison d'une quantité d'enzyme présentant une activité de 0,1-12 unités Anson (AU) pour 100 g de matière sèche de substrat.

On réalise la première étape d'hydrolyse durant 10-60 min tout en ajustant le pH et la température à des valeurs favorables à l'activité de l'enzyme, et l'on réalise la seconde étape d'hydrolyse durant 1-8 h en ajustant la température à une valeur égale ou supérieure, notamment de 0-10°C, à la température de la première étape.

On peut prévoir des étapes intermédiaires ou complémentaires, notamment une étape de mélange préliminaire, de préférence en tube garni d'éléments mélangeurs statiques; une étape de dénaturation thermique, avant, au milieu de ou après la première étape d'hydrolyse, notamment à l'aide d'un échangeur de chaleur ou d'un tube garni d'éléments mélangeurs statiques; une ou plusieurs étapes d'inactivation d'enzyme, en particulier après la deuxième étape d'hydrolyse, notamment à l'aide d'un échangeur de chaleur et/ou d'un dispositif à injection de vapeur et/ou d'un tube garni d'éléments mélangeurs statiques; et/ou une étape de refroidissement, en particulier après une étape de dénaturation, notamment à l'aide d'un échangeur de chaleur ou de préférence en tube garni d'éléments mélangeurs statiques, par exemple.

On peut inactiver l'enzyme en une, ou de préférence en deux étapes, une première étape correspondant plus précisément à une autodigestion de l'enzyme et une seconde étape correspondant plus précisément à une stérilisation.

On peut subdiviser ladite deuxième étape d'hydrolyse en tube en au moins deux parties réalisées en au moins deux tubes branchés en série. Dans ce cas, on peut réaliser un ajustement de pH et/ou ajouter de l'enzyme entre deux tubes successifs.

Pour le ou les ajustement du pH, on utilise de préférence un réactif adéquat, soit alcalin, tel que KOH, NaOH ou Ca(OH)₂, soit acide, tel que HCl ou HPO₄, par exemple.

Dans une forme de réalisation préférée du présent procédé, on choisit comme enzyme une protéase alcaline bactérienne, notamment celle produite par Bacillus licheniformis et commercialisée par la firme Novo sous la désignation "alcalase", en particulier "alcalase 0.6 L" ou "alcalase 2.4 L", par exemple.

On a constaté qu'avec cette forme de réalisation préférée on pouvait obtenir un hydrolysat présentant un NPN particulièrement élevé et une allergénicité particulièrement réduite.

Pour ce faire, on peut réaliser ladite première étape d'hydrolyse à un pH de 7,0-10,0 à 50-80°C, de préférence 63-73°C, et la seconde étape d'hydrolyse à un pH de 6,5-8,0 à 55-80°C, de préférence 65-73°C. On peut prévoir, soit avant la première étape d'hydrolyse, soit entre lesdites deux étapes d'hydrolyse, une étape de dénaturation thermique à 80-120°C, de préférence à 85-95°C, durant 30 s à 10 min, de préférence durant 4-6 min. On peut ensuite inactiver l'enzyme par une étape d'autodigestion à 70-110°C, de préférence 85-90°C, durant 10 s à 20 min, de préférence durant 2-8 min, suivie d'une étape de stérilisation à 110-150°C, de préférence 120-130°C, durant 5 s à 5 min, de préférence durant 30 s à 2 min.

Dans une autre forme de réalisation préférée du présent procédé, on choisit comme enzyme une combinaison comprenant d'une part une protéase alcaline bactérienne, notamment celle produite par Bacillus licheniformis et commercialisée par la firme Novo sous la désignation "alcalase", en particulier "alcalase 0.6 L" ou "alcalase 2.4 L", et d'autre part une enzyme pancréatique, notamment la trypsine, par exemple.

Dans cette autre forme de réalisation préférée, on peut soumettre séparément deux substrats, notamment deux substrats lactosériques, chacun à une hydrolyse distincte, avec l'une de ces deux enzymes, selon le présent procédé, jusqu'à une étape commune, de préférence jusqu'à une étape commune de stérilisation suivant deux étapes distinctes d'autodigestion des deux enzymes différentes. On peut également soumettre successivement le même substrat à l'action de l'une puis de l'autre de ces deux enzymes. On a constaté en effet qu'un produit d'hydrolyse des protéines du lactoserum, par exemple, obtenu par cette combinaison peut présenter une stabilité à la conservation particulièrement bonne.

Pour la mise en oeuvre du présent procédé avec une enzyme pancréatique, en particulier la trypsine, par exemple, notamment dans le cadre de la combinaison ci-dessus, on peut avantageusement utiliser les conditions de pH et de température décrites dans EP 322589.

L'appareil pour la mise en oeuvre du procédé selon la présente invention comprend donc une cuve d'hydrolyse à double manteau avec agitateur relié en amont à un dispositif doseur de substrat et à un dispositif doseur d'enzyme, et relié en aval à au moins un tube d'hydrolyse garni d'éléments mélangeurs statiques.

Dans cet appareil, ledit tube peut être disposé verticalement, son extrémité inférieure étant reliée à ladite cuve et son extrémité supérieure débouchant sur une conduite de sortie. Il peut aussi être disposé horizontalement ou dans toute autre position. Il présente de préférence une longueur supérieure à quatre fois son diamètre.

De préférence également, lesdits dispositifs doseur de substrat et doseur d'enzyme comportent chacun un récipient d'alimentation relié à ladite cuve d'hydrolyse par l'intermédiaire d'une pompe volumétrique.

L'appareil peut comprendre en outre un dispositif doseur de réactif comportant un récipient d'alimentation relié à la cuve d'hydrolyse par l'intermédiaire d'une pompe volumétrique commandée par un pH mètre.

L'appareil peut également comprendre plusieurs tubes d'hydrolyse garnis d'éléments mélangeurs statiques, branchés en série en aval de la cuve par des conduites de liaison qui peuvent être reliées en amont au dispositif doseur d'enzyme et au dispositif doseur de réactif.

On peut également prévoir un tube garni d'éléments mélangeurs statiques entre lesdits dispositifs doseur d'enzyme, doseur de substrat et/ou doseur de réactif et ladite cuve.

L'appareil pour la mise en oeuvre du procédé selon la présente invention est décrit ci-après en référence au dessin annexé qui en illustre, à titre d'exemple, deux formes de réalisation. Dans ce dessin,
- la Figure 1 représente schématiquement une première forme de réalisation de l'appareil, comprenant une cuve et un tube garni d'éléments mélangeurs statiques,
- la Figure 2 représente schématiquement une deuxième forme de réalisation de l'appareil, comprenant une cuve et plusieurs tubes d'hydrolyse garnis d'éléments mélangeurs statiques, et

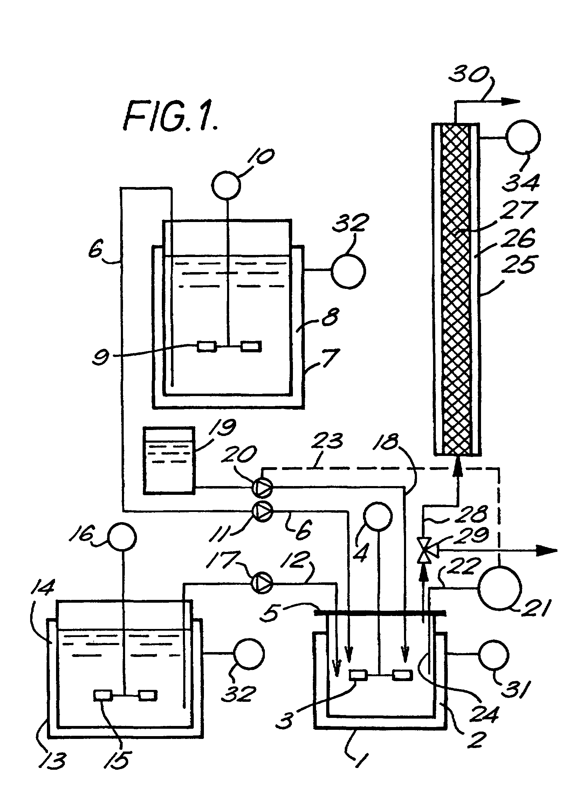

Comme on le voit à la Figure 1, le présent appareil comprend une cuve d'hydrolyse 1 à double manteau 2 avec agitateur 3 entraîné par un moteur 4. Cette cuve est fermée de manière étanche par un couvercle 5 au travers duquel passent diverses conduites et l'axe de l'agitateur 3.

La cuve d'hydrolyse 1 est reliée en amont par une conduite 6 à un dispositif doseur de substrat 7-11, par une conduite 12 à un dispositif doseur d'enzyme 13-17, et par une conduite 18 à un dispositif doseur de réactif 19-24.

Le dispositif doseur de substrat comporte un récipient d'alimentation en substrat 7 à double manteau 8 et agitateur 9 entraîné par un moteur 10. Le récipient 7 est relié à la cuve d'hydrolyse 1 par l'intermédiaire de la pompe volumétrique 11 branchée sur la conduite 6.

Le dispositif doseur d'enzyme comporte un récipient d'alimentation en enzyme 13 à double manteau 14 et agitateur 15 entraîné par un moteur 16. Le récipient 13 est relié à la cuve d'hydrolyse 1 par l'intermédiaire de la pompe volumétrique 17 branchée sur la conduite 12.

Le dispositif doseur de réactif comporte un récipient d'alimentation en réactif 19 relié à la cuve d'hydrolyse 1 par l'intermédiaire d'une pompe volumétrique 20 branchée sur la conduite 18. Cette pompe volumétrique 18 est commandée par un pH-mètre 21 dont l'électrode de mesure 24 plonge dans la cuve 1 à travers le couvercle 5 et qui est relié électriquement (ligne hachurée 23) à un dispositif électronique de commande de la pompe 20 non représenté.

La cuve d'hydrolyse 1 est reliée en aval à un tube d'hydrolyse 25 à double manteau 26 garni d'éléments mélangeurs statiques 27 constitués de croisillons métalliques ou en plastique imbriqués les uns dans les autres. La cuve 1 est reliée au tube 25 par une conduite sur laquelle est branchée une vanne à trois voies 29 destinée à permettre des prélèvements d'hydrolysat dans la cuve.

Le tube 25 est disposé verticalement, son extrémité inférieure étant reliée à la cuve 1 et son extrémité supérieure débouchant sur une conduite de sortie 30.

La température d'un fluide circulant dans chacun des doubles manteaux est régulée par un dispositif représenté symboliquement en 31 pour la cuve 1, 32 pour le récipient 7, 33 pour le récipient 13, et 34 pour le tube 25.

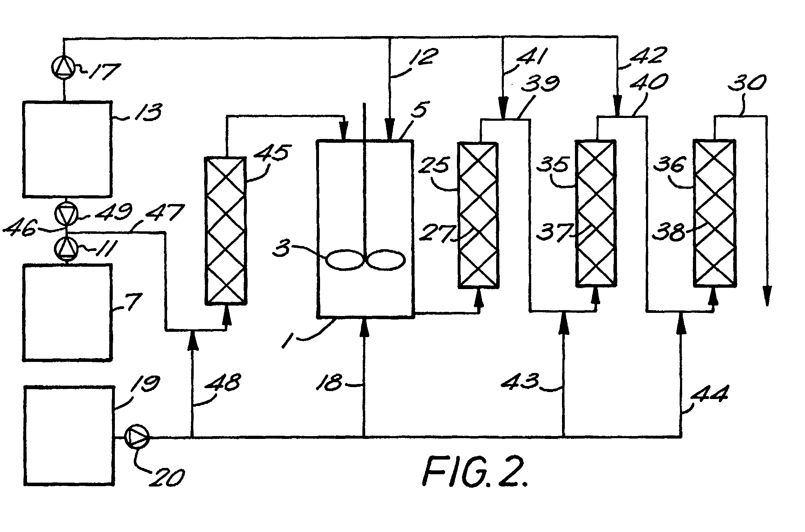

A la Figure 2, les éléments de cette deuxième forme de réalisation de l'appareil qui correspondent aux éléments de la première forme de réalisation selon la Figure 1 y sont désignés par les mêmes chiffres de référence.

Dans cette deuxième forme de réalisation, l'appareil comprend plusieurs tubes d'hydrolyse 25, 35, 36 garnis d'éléments mélangeurs statiques 27, 37, 38 et branchés en série en aval de la cuve 1 par des conduites de liaison 39, 40 reliées en amont au récipient d'alimentation en enzyme 13 par des conduites 41, 42 qui rejoignent la conduite 12 sur laquelle est branchée la pompe volumétrique 17.

Ces conduites de liaison 39, 40 sont également reliées en amont au récipient d'alimentation en réactif 19 par des conduites 43, 44 qui rejoignent la conduite 18 sur laquelle est branchée la pompe volumétrique 20.

Dans cette deuxième forme de réalisation du présent appareil, on a également prévu un tube de mélange 45 garni d'éléments mélangeurs statiques entre les dispositifs doseur d'enzyme, doseur de substrat et doseur de réactif et la cuve 1.

Les différents récipients d'alimentation en enzyme, en substrat et en réactif sont reliés à ce tube 45 par des conduites 46, 47, 48 sur lesquelles sont respectivement branchés une pompe volumétrique 49 et les pompes volumétriques 11 et 20.

Les exemples ci-après sont présentés à titre d'illustration du procédé selon la présente invention. Les pourcentages et parties y sont donnés en poids.

### Exemple 1

On met en oeuvre le présent procédé à l'aide d'un appareil semblable à celui décrit en référence à la Figure 1, dans lequel la cuve d'hydrolyse présente un volume de 30 1 et le tube d'hydrolyse garni d'éléments mélangeurs statiques présente un volume de 180 1 pour une hauteur de 3 m.

On utilise comme substrat un concentrat de protéines de lactosérum partiellement déminéralisé présentant une teneur en matière sèche de 20% et des teneurs respectives, en % sur matière sèche, de environ 23% de protéines, 1,9% de matière grasse, 73% de lactose et 1,3% de cendres.

On utilise comme enzyme de la trypsine porcine présentant une activité de 3 AU/g, à raison de 1 g d'enzyme pour 100 g de matière sèche du substrat, soit 3 AU pour 100 g de matière sèche du substrat.

On utilise comme réactif du KOH 2N.

On remplit tout d'abord la cuve de substrat, on y mélange l'enzyme et l'on démarre le processus d'hydrolyse en discontinu à pH 7,3 à 60°C durant 15 min, après quoi le substrat hydrolysé présente un NPN de 40%.

On poursuit alors le processus en continu à un débit tel que le temps de séjour moyen du substrat dans la cuve soit de 30 min et le temps de séjour de l'hydrolysat dans le tube soit de 3 h. On maintient dans la cuve une température de 60°C et un pH de 7,3. On maintient dans le tube une température de 60°C et on y laisse le pH flotter, de sorte qu'il diminue spontanément de environ 7,3 à l'entrée à environ 6,9 à la sortie.

L'hydrolysat présente un NPN de 65% au sortir du tube.

Si, pour comparaison, on réalise une hydrolyse du même substrat, avec la même enzyme, dans le même rapport enzyme-substrat, en discontinu dans une cuve de 200 l, à pH 7,3 à 60°C durant environ 7 h, on obtient un hydrolysat présentant un NPN de 60%.

### Exemple 2

On procède de manière semblable à celle décrite à l'exemple 1, à l'exception du fait que lors de trois essais distincts on varie le volume utile de la cuve de manière que le NPN obtenu après le passage du substrat dans la cuve soit respectivement de 15, 35 et 45%.

On obtient ainsi à la sortie du tube des hydrolysats présentant des NPN respectifs de 59, 63 et 66%.

Pour comparaison, dans les mêmes conditions en discontinu en cuve, à savoir à pH 7,3 à 60°C avec un substrat à 20% de matière sèche et une quantité d'enzyme présentant une activité de 3 AU pour 100 g de matière sèche du substrat, on obtient en environ 7 h un NPN de 60%.

En d'autres termes, avec le présent procédé, on obtient en continu un NPN supérieur à celui qu'on obtiendrait en discontinu dès que le substrat présente un NPN supérieur à environ 35% à l'entrée du tube.

### Exemple 3

On procède de manière semblable à celle décrite à l'exemple 1, à l'exception du fait que l'on maintient dans la cuve un pH de 7,8 et une température de 55°C au lieu de pH 7,3 et 60°C.

On obtient à la sortie du tube un hydrolysat présentant un NPN de 70%.

### Exemple 4

On met en oeuvre le présent procédé à l'aide d'un appareil de type semblable à celui décrit en référence à la Figure 2.

On utilise comme substrat un concentrat de protéines de lactosérum présentant une teneur en matière sèche de 33% dont 7,5% de protéines.

On utilise comme enzyme une protéase alcaline bactérienne produite par Bacillus licheniformis et commercialisée par la firme Novo sous la désignation "alcalase 2.4 L", qui présente une activité de 2,4 AU/g. On utilise cette enzyme à raison d'une quantité totale de 4-8,6% sur protéine, soit 2,2-4,7 AU pour 100 g de matière sèche du substrat.

On utilise comme réactif du KOH 2N.

Après avoir démarré le processus de manière adéquate, on le poursuit en continu. Le débit de substrat et les dimensions des tubes et de la cuve sont déterminés en sorte que les temps de séjour du substrat ou de l'hydrolysat soient respectivement de 5-10 min dans un tube de mélange préliminaire garni d'éléments mélangeurs statiques précédant la cuve, de 5-8 min dans un tube de dénaturation thermique garni d'éléments mélangeurs statiques branché en série entre le tube de mélange préliminaire et la cuve, de 25-40 min dans la cuve (1ère étape d'hydrolyse), de 15-25 min dans un premier tube A garni d'éléments mélangeurs statiques suivant la cuve (tube A de la 2ème étape d'hydrolyse), de 15-25 min dans un deuxième tube B garni d'éléments mélangeurs statiques (tube B de la 2ème étape d'hydrolyse), de 0-100 min dans un troisième tube C garni d'éléments mélangeurs statiques (tube C de la 2ème étape d'hydrolyse), de 5-20 min dans un tube d'inactivation garni d'éléments mélangeurs statiques branché en série à la suite du tube C, et de 5-15 min dans un tube de refroidissemnt garni d'éléments mélangeurs statiques.

On répartit ladite quantité totale d'enzyme en quatre parts, à savoir une première part de 5-15% du total mélangée au substrat dans le tube de mélange préliminaire, une deuxième part de 30-40% du total mélangée au substrat dans la cuve, une troisième part de 20-30% du total mélangée au substrat dans ledit tube A et une quatrième part de 20-30% du total mélangée au substrat dans ledit tube B.

On ajuste le pH du substrat à 7,3 jusqu'au dit tube B à partir duquel on laisse le pH flotter.

On ajuste la température à 75°C dans le tube de mélange préliminaire, 85°C dans le tube de dénaturation thermique, 70°C dans la cuve, 71°C dans le tube A et le tube B, 80-105°C dans le tube d'inactivation et 2-8°C dans le tube de refroidissement.

On recueille l'hydrolysat ainsi produit après le tube de refroidissement.

### Exemple 5

On met en oeuvre le présent procédé à l'aide d'un appareil semblable à celui décrit en référence à la Figure 1, dans lequel la cuve d'hydrolyse présente un volume de 2,8 1 et le tube d'hydrolyse garni d'éléments mélangeurs statiques présente un volume de 11,6 1 pour une longueur de environ 5 m.

On utilise comme substrat un concentrat de protéines de lactosérum présentant une teneur en matière sèche de 33% dont 7,5% de protéines.

On utilise comme enzyme l'alcalase 2.4 L, à raison d'une quantité totale de 6,3% sur protéine, soit 3,4 AU pour 100 g de matière sèche du substrat.

On utilise comme réactif du KOH 2N.

On remplit tout d'abord la cuve de substrat, on y mélange l'enzyme et l'on démarre le processus d'hydrolyse en discontinu à pH 7,3 à 70°C durant 25 min.

On poursuit alors le processus en continu à un débit tel que le temps de séjour total de l'hydrolysat dans l'appareil soit de 240 min (47 min dans la cuve et 193 min dans le tube). On maintient dans la cuve une température de 70°C et un pH de 7,3. On maintient dans le tube une température de 70°C et on y laisse le pH flotter, de sorte qu'il diminue spontanément de environ 7,3 à l'entrée à environ 6,72 à la sortie.

On prélève et analyse des échantillons à la sortie du tube aux temps 0, 60, 120, et 180 min à compter de 240 min après la mise en route du processus en continu. Ces échantillons présentent les pH et teneurs en azote aminé indiqués dans le tableau I ci-après où l'on a également reporté la quantité correspondante de KOH utilisée pour maintenir le pH à 7,3 dans la cuve.

**Tableau I**

| Temps (min) | pH | azote aminé (%) | KOH (g/h) |
|---|---|---|---|
| 0 | 6,71 | 0,26 | 124 |
| 60 | 6,72 | 0,25 | 123 |
| 120 | 6,73 | 0,26 | 125 |
| 180 | 6,71 | 0,26 | 125 |

Les quantités de KOH indiquées en g/h correspondent à une consommation moyenne de 44,375 g par l de la cuve pour un temps de résidence de 47 min.

On constate sur ce tableau I que les caractéristiques présentées par l'hydrolysat ne varient pratiquement pas, quel que soit le moment où on en prélève et analyse des échantillons à la sortie du tube. On vérifie également par électrophorèse de zone en gel de polyacrylamide (méthode SDS-Page) que le profil peptidique avantageux de ces échantillons, majorité de petits peptides, reste également remarquablement constant.

Pour comparaison, on soumet le même substrat à une hydrolyse enzymatique avec la même enzyme, dans le même rapport enzyme:substrat, en discontinu dans une cuve de 2 l à 70°C à pH maintenu à 7,3 durant 47 min. Après ces premières 47 min, on laisse le pH flotter. On prélève et analyse des échantillons au temps 47 min à compter du début de l'hydrolyse, puis à différents temps jusqu'à et au-delà de 240 min. Ces échantillons présentent les pH et teneurs en azote aminé indiqués dans le tableau II ci-après.

**Tableau II**

| Temps (min) | pH | azote aminé (%) |
|---|---|---|
| 47 | 7,30 | 0,21 |
| 67 | 7,0 | 0,22 |
| 140 | 6,77 | 0,24 |
| 197 | 6,75 | 0,27 |
| 240 | 6,72 | 0,26 |
| 300 | 6,68 | |
| 360 | 6,65 | |

La quantité de KOH utilisée pour maintenir le pH à 7,3 durant les premières 47 min est de 45,5 g par l de la cuve.

On constate sur ce tableau II que les caractéristiques présentées par l'hydrolysat obtenu en discontinu en cuve varient rapidement, également après le temps de 240 min correspondant au temps de séjour en continu dans l'appareil utilisé au présent exemple 6.

Ceci démontre l'un des avantages du présent procédé dans lequel aucune évolution du produit ne doit être crainte qui soit comparable à celle qui se produit durant le temps nécessaire à la vidange de la cuve dans un procédé en discontinu.

### Exemple 6

On met en oeuvre le présent procédé à l'aide d'un appareil semblable à celui décrit en référence à la Figure 1, dans lequel la cuve d'hydrolyse présente un volume de 5 1 et le tube d'hydrolyse garni d'éléments mélangeurs statiques présente un volume de 9,6 l pour une longueur de environ 5 m.

On utilise comme substrat un concentrat de protéines de lactosérum présentant une teneur en matière sèche de 33% dont 7,5% de protéines.

On utilise comme enzyme l'alcalase 2.4 L, à raison d'une quantité totale de 8% sur protéine, soit 4,4 AU pour 100 g de matière sèche du substrat. De ces 8%, 2% sont utilisés dans la cuve et 6% sont ajoutés à l'entrée du tube.

On utilise comme réactif du KOH 2N.

Au cours de deux essais distincts, on remplit tout d'abord la cuve de substrat, on y mélange l'enzyme et l'on démarre le processus d'hydrolyse en discontinu à pH 7,3 à deux températures différentes, 72,5 et 74°C, durant 40 min.

On poursuit alors chaque processus en continu à un débit tel que le temps de séjour total de l'hydrolysat dans l'appareil soit de 116 min (40 min dans la cuve et 76 min dans le tube). On maintient dans la cuve des températures respectives de 72,5°C et 74°C et un pH de 7,3 pour chacun des deux essais. On maintient dans le tube une température de 72°C et on y laisse le pH flotter.

Les hydrolysats ainsi obtenus, correspondant aux températures de 72,5°C et 74°C dans la cuve, présentent des NPN respectifs de 97,2 % et 91,4 % et ont nécessité l'usage de quantités respectives de 205 g/h et 198 g/h de KOH pour maintenir le pH à 7,3 dans la cuve. On observe en outre par électrophorèse de zone en gel de polyacrylamide (méthode SDS-Page) qu'ils présentent un profil peptidique relativement étroit.

### Exemple comparatif (i)

On soumet un concentrat de protéines de lactosérum présentant une teneur en matière sèche de 33% dont 7,5% de protéines à une hydrolyse enzymatique avec l'alcalase 2.4 L, à raison d'une quantité totale de 4% sur protéine, soit 2,2 AU pour 100 g de matière sèche du substrat, en continu dans une cuve de 5 l à 70°C durant 200 min à des pH respectifs de 6,4, 6,8, 7,3 et 7,8 au cours de quatre essais distincts.

Les hydrolysats ainsi obtenus présentent des NPN compris entre 80 et 83% et ont nécessité l'usage de quantités respectives de KOH, en g/h, de 33,4, 50,1, 60,2 et 77,2 pour maintenir leur pH à 6,4, 6,8, 7,3 et 7,8. Ils présentent en outre des teneurs respectives en azote aminé de 0,17, 0,20, 0,21 et 0,22%. On observe en outre par test SDS-Page quils présentent un profil peptidique relativement large.

### Exemple 7

On met en oeuvre le présent procédé à l'aide d'un appareil semblable à celui décrit en référence à la Figure 1, dans lequel la cuve d'hydrolyse présente un volume de 2,8 l et le tube d'hydrolyse garni d'éléments mélangeurs statiques présente un volume de 11,6 l pour une longueur de environ 5 m.

On utilise comme substrat un concentrat de protéines de lactosérum présentant une teneur en matière sèche de 33% dont 7,5% de protéines.

On utilise comme enzyme l'alcalase 2.4 L, à raison d'une quantité totale de 7% sur protéine, soit 3,8 AU pour 100 g de matière sèche du substrat. De ces 7%, 2% sont utilisés dans la cuve et 5% sont ajoutés à l'entrée du tube.

On utilise comme réactif du KOH 2N.

On remplit tout d'abord la cuve de substrat, on y mélange l'enzyme et l'on démarre le processus d'hydrolyse en discontinu à pH 7,8 à 70°C durant 25 min.

On poursuit alors le processus en continu à un débit tel que le temps de séjour de l'hydrolysat soit de 45 min dans la cuve et 170 min dans le tube d'hydrolyse, au total 215 min. On maintient dans la cuve une température de 70°C et un pH de 7,8. On maintient dans le tube une température de 70°C et on y laisse le pH flotter, de sorte qu'il diminue spontanément de environ 7,8 à l'entrée à environ 6,67 à la sortie.

Dans un tube d'inactivation garni d'éléments mélangeurs statiques branché en série à la sortie du tube d'hydrolyse, on inactive l'hydrolysat durant 18 min à 90°C. Dans un tube de refroidissement garni d'éléments mélangeurs statiques branché en série en aval du tube d'inactivation , on refroidit l'hydrolysat à température ambiante.

A la sortie du tube de refroidissement, on prélève et analyse des échantillons correspondants aux temps 0, 60, 120, 180 et 240 min comptés à la sortie du tube d'hydrolyse à partir de 215 min après la mise en route du processus en continu. Ces échantillons présentent les pH, teneurs en azote aminé, blocages de lysine et NPN indiqués dans le tableau III ci-après où l'on a également reporté la quantité correspondante de KOH utilisée pour maintenir le pH à 7,8 dans la cuve.

**Tableau III**

| Temps (min) | pH | azote aminé (%) | KOH (g/h) | blocage lysine (%) | NPN (%) |
|---|---|---|---|---|---|
| 0 | 6,67 | 0,26 | 125 | 16,3 | 95 |
| 60 | 6,68 | 0,25 | 124 | 16,2 | 96 |
| 120 | 6,67 | 0,26 | 128 | 16,3 | 94 |
| 180 | 6,67 | 0,27 | 124 | 16,2 | 95 |
| 240 | 6,67 | 0,26 | 127 | 16,1 | 96 |

On constate sur ce tableau III, comme on le voit également sur le tableau I de l'exemple 5, que les caractéristiques présentées par l'hydrolysat ne varient pratiquement pas, quel que soit le moment où on en prélève et analyse des échantillons à la sortie du tube d'hydrolyse.

On examine également le profil peptidique et les propriétés hypoallergéniques du produit obtenu dans les conditions selon le présent exemple, en le soumettant à des tests HPLC, ELISA et sérotonine-³H dont les résultats sont présentés plus loin aux tableaux V, VI et VII.

Pour comparaison, on soumet 160 kg du même substrat à une hydrolyse enzymatique avec la même enzyme, à raison d'une quantité totale de 7% sur protéine, en discontinu dans une cuve, à 70°C. De ces 7% d'enzyme, 2% sont utilisés pour une première partie de l'hydrolyse à pH 7,8 durant 45 min après lesquelles on laisse le pH flotter. Après 60 min on ajoute les 5% d'enzyme restants et l'on continue l'hydrolyse à 70°C et à pH flottant jusqu'à 215 min et au-delà.

On soutire 20 kg d'hydrolysat après 120 min à compter du début de l'hydrolyse. On fait de même après 150, 180, 200, 250, 300 et 360 min. En cours de route, on prélève un échantillon pour analyse après 215 min.

Les hydrolysats correspondant aux divers prélèvements et échantillons sont immédiatement inactivés (dans un échangeur de chaleur, 18 min à 90°C), puis refroidis à température ambiante (dans un échangeur de chaleur), et analysés. Ils présentent les pH, teneurs en azote aminé (% sur poudre à 97% de matière sèche), blocage de lysine et NPN indiqués dans le tableau IV ci-après.

**Tableau IV**

| Temps (min) | pH | azote aminé (%, poudre) | blocage lysine (%) | NPN (%) |
|---|---|---|---|---|
| 60 | 7,56 | | | |
| 120 | 6,97 | 0,60 | 15,3 | |
| 150 | 6,87 | 0,63 | 17,2 | 90 |
| 180 | 6,82 | 0,66 | 18,2 | 94 |
| 200 | 6,80 | 0,68 | 18,3 | 95 |
| 215 | 6,80 | 0,68 | 18,9 | 95 |
| 250 | 6,79 | 0,69 | 19,4 | 96 |
| 300 | 6,77 | 0,71 | 19,5 | 96 |
| 360 | 6.76 | 0,74 | 19,6 | 97 |

On constate sur ce tableau IV, comme on l'avait fait sur le tableau II de l'exemple 5, que les caractéristiques présentées par l'hydrolysat obtenu en discontinu en cuve varient rapidement, également après les 215 min correspondant au temps de séjour en continu dans l'appareil utilisé au présent exemple 8.

Ceci confirme l'un des avantages du présent procédé dans lequel aucune évolution du produit ne doit être crainte qui soit comparable à celle qui se produit durant le temps nécessaire à la vidange de la cuve dans un un procédé en discontinu.

On examine également le profil peptidique et les propriétés hypoallergéniques du produit obtenu dans les conditions selon l'exemple comparatif ci-dessus, au temps 215 min, en le soumettant à des tests HPLC, ELISA et sérotonine-³H dont les résultats sont présentés ci-après aux tableaux V,VI et VII.

On réalise des tests analogues sur le produit obtenu en cuve en continu dans les conditions correspondant à pH 7,3 présentées à l'exemple comparatif (i) et on les présente également dans les tableaux V, VI et VII ci-après.

**Tableau V**

| Profil peptidique (test HPLC) | | | | | |
|---|---|---|---|---|---|
| Produit selon | Pourcents de peptides compris dans les domaines compris dans les limites de poids moléculaires exprimées en kDalton | | | | |
| | >14 | 14-6 | 6-3,5 | 3,5-1,0 | < 1 |
| Exemple 7 | 5 | 7 | 9 | 30 | 49 |
| Comparaison (cuve disc) | 4 | 7 | 10 | 31 | 48 |
| Exemple comparatif (i) | 21 | 13 | 9 | 24 | 33 |

Les résultats des tests selon ce Tableau V illustrent bien le fait qu'un hydrolysat obtenu en continu par le procédé selon la présente invention peut présenter un profil peptidique au moins aussi étroit et aussi centré sur les petits peptides qu'un hydrolysat obtenu pour comparaison en cuve en discontinu.

**Tableau VI**

| Test d'inhibition ELISA | | | |
|---|---|---|---|
| Produit selon | Antigénicité résiduelle exprimée en ug d'antigène par g de protéine | | |
| | BLG | BSA | CAS |
| Exemple 7 | 53 | 20 | 150 |
| Comparaison (cuve disc) | 41 | 7 | 141 |
| Exemple comparatif (i) | 111 | > 1000 | 319 |

**Tableau VII**

| Test de relâchement de sérotonine-³H | |
|---|---|
| Produit selon | Antigénicité résiduelle exprimée en ug d'équivalent BLG pour le relâchement par g d'équivalent protéine |
| Exemple 7 | 20 |
| Comparaison (cuve disc) | 5 |
| Exemple comparatif (i) | 50 |

Les résultats dès tests selon ces Tableaux VI et VII illustrent le fait qu'un hydrolysat obtenu par le procédé selon la présente invention peut être au moins autant hypoallergénique qu'un hydrolysat obtenu pour comparaison en cuve en discontinu.

### Exemple 8

On met en oeuvre le présent procédé de la manière décrite à l'exemple 6 dans les conditions correspondant à une température de 72,5°C dans la cuve. Le tube est subdivisé en neuf segments. Des échantillons sont prélevés entre deux segments successifs au fur et à mesure que l'hydrolyse en continu progresse après le démarrage en discontinu. Des échantillons sont prélevés à la sortie du tube au même rythme, dès que la durée de l'hydrolyse en continu atteint le temps correspondant au temps de résidence du produit dans le tube.

On détermine la teneur en azote aminé des échantillons. On divise chacune de ces teneurs par la teneur d'équilibre vers laquelle tend l'hydrolysat. Sur un système de coordonnées, on reporte en ordonnée les quotients obtenus et en abscisse les quotients des temps de prélèvement des échantillons divisés par le temps de séjour de l'hydrolysat dans l'appareil.

On obtient une courbe sigmoïde croissant à partir de l'abscisse 0,8, coupant la verticale de l'abscisse 1,0 aux deux tiers de sa valeur maximale et atteignant sa valeur maximale, autrement dit touchant l'horizontale de l'ordonnée 1,0 à la verticale de l'abscisse 1,2.

Pour comparaison, on réalise le même essai, à l'exception du fait que l'on utilise un tube vide présentant par ailleurs les mêmes dimensions que le tube garni d'éléments mélangeurs statiques.

On prélève des échantillons dans les mêmes conditions, on établit les mêmes quotients et l'on trace la courbe correspondante de la même manière.

On obtient une courbe sigmoïde croissant à partir de l'abscisse 0,6, coupant la verticale de l'abscisse 1,0 à la moitié de sa valeur maximale et n'atteignant sa valeur maximale, à savoir 1,0, qu'au delà de la verticale de l'abscisse 1,8.

Ceci démontre deux autres avantages encore du présent procédé, à savoir d'une part la rapidité avec laquelle un régime stationnaire peut être atteint, et d'autre part l'homogénéité que présente le présent hydrolysat à la sortie du présent appareil.

## Revendications

1. Procédé d'hydrolyse enzymatique de protéines, dans lequel on soumet un substrat protéique à une hydrolyse en continu avec une enzyme protéolytique, on réalise une première étape d'hydrolyse enzymatique relativement courte en cuve sous agitation et une deuxième étape d'hydrolyse enzymatique relativement longue en tube, on réalise la première étape durant 10-60 min tout en ajustant le pH et la température à des valeurs favorables à l'activité de l'enzyme, on réalise la seconde étape durant 1-8 h en ajustant la température à une valeur égale ou supérieure à la température de la première étape, et l'on réalise la deuxième étape d'hydrolyse en tube garni d'éléments mélangeurs statiques.

2. Procédé selon la revendication 1, dans lequel ledit substrat est une matière première alimentaire riche en protéines, notamment des farines ou semoules de graines ou de tourteaux d'oléagineuses, des levures ou bactéries alimentaires, de la chair animale ou de poisson hachée, ou des laits ou dérivés du lait, sous forme de particules en suspension aqueuse ou de suspension aqueuse.

3. Procédé selon la revendication 1, dans lequel ledit substrat protéique est un substrat lactosérique contenant les protéines du lactosérum, notamment un lactosérum doux de fromagerie ou un lactosérum acide de caséinerie, tel quel ou sous forme déminéralisée ou délactosée, liquide ou reconstitué.

4. Procédé selon la revendication 1, dans lequel l'enzyme protéolytique est choisie dans le groupe formé par la trypsine, la chymotrypsine, la pancréatine, les protéases bactériennes, les protéases fongiques et leurs mélanges.

5. Procédé selon la revendication 1, dans lequel on mélange l'enzyme protéolytique et le substrat à raison d'une quantité d'enzyme présentant une activité de 0,1-12 AU pour 100 g de matière sèche du substrat.

6. Procédé selon la revendication 1, dans lequel on réalise la seconde étape en ajustant la température à une valeur égale ou supérieure de 0-10°C à la température de la première étape.

7. Appareil pour la mise en oeuvre du procédé selon la revendication 1, comprenant une cuve d'hydrolyse avec agitateur reliée en amont à un dispositif doseur de substrat et à un dispositif doseur d'enzyme, et reliée en aval à au moins un tube d'hydrolyse, ledit tube d'hydrolyse étant garni d'éléments mélangeurs statiques et étant de relativement grande dimension, et la cuve étant de relativement faible dimension.

8. Appareil selon la revendication 7, dans lequel ledit tube est disposé verticalement, son extrémité inférieure étant reliée à ladite cuve et son extrémité supérieure débouchant sur une conduite de sortie.

9. Appareil selon la revendication 7, dans lequel lesdits dispositifs doseur de substrat et doseur d'enzyme comportent chacun un récipient d'alimentation relié à ladite cuve d'hydrolyse.

10. Appareil selon la revendication 9, comprenant en outre un dispositif doseur de réactif comportant un récipient d'alimentation relié à la cuve d'hydrolyse par l'intermédiaire d'une pompe volumétrique commandée par un pH-mètre branché sur la cuve.

11. Appareil selon la revendication 10, comprenant plusieurs tubes d'hydrolyse garnis d'éléments mélangeurs statiques, branchés en série en aval de la cuve par l'intermédiaire de conduites de liaison reliées en amont au dispositif doseur d'enzyme et au dispositif doseur de réactif.

## Patentansprüche

1. Verfahren zur enzymatischen Hydrolyse von Proteinen, bei dem man ein Proteinsubstrat einer kontinuierlichen Hydrolyse mit einem proteolytischen Enzym unterzieht, einen ersten, relativ kurzen Schritt der enzymatischen Hydrolyse im Behälter unter Rühren und einen zweiten, relativ langen Schritt der enzymatischen Hydrolyse im Rohr durchführt, den ersten Schritt während 10-60 min durchführt, indem man den pH-Wert und die Temperatur auf Werte einstellt, die für die Aktivität des Enzyms günstig sind, den zweiten Schritt während 1-8 h durchführt, indem man die Temperatur auf einen Wert einstellt, der gleich der Temperatur des ersten Schritts oder höher ist, und den zweiten Hydrolyseschritt in einem mit statischen Mischerelementen ausgerüsteten Rohr vornimmt.

2. Verfahren nach Anspruch 1, bei dem das Substrat ein an Proteinen reiches Nahrungsmittelausgangsmaterial ist, insbesondere Mehl oder Grieß von Kernen oder von Presskuchen von Ölpflanzen, Hefen oder Nahrungsmittelbakterien, Tierfleisch oder gehackter Fisch oder Milch oder Milchderivate in Form von Teilchen in wässriger Suspension oder von wässriger Suspension.

3. Verfahren nach Anspruch 1, bei dem das Proteinsubstrat ein Molkesubstrat ist, das Molkeproteine enthält, insbesondere Süßmolke der Käseherstellung oder Sauermolke der Kaseinherstellung, so, wie SIE ist, oder in entmineralisierter oder von Lactose befreiter, flüssiger oder rekonstituierter Form.

4. Verfahren nach Anspruch 1, bei dem das proteolytische Enzym aus der Gruppe ausgewählt ist, die aus Trypsin, Chymotrypsin, Pancreatin, bakteriellen Proteasen, Pilzproteasen und ihren Mischungen besteht.

5. Verfahren nach Anspruch 1, bei dem man das proteolytische Enzym und das Substrat in einem Verhältnis von einer Enzymmenge mit einer Aktivität von 0,1-12 AU auf 100 g Trockenmasse des Substrats mischt.

6. Verfahren nach Anspruch 1, bei dem man den zweiten Schritt ausführt, indem man die Temperatur auf einen Wert einstellt, der gleich der Temperatur des ersten Schritts oder um 0-10°C höher ist.

7. Vorrichtung zur Durchführung des Verfahrens nach Anspruch 1, die einen Hydrolysebehälter mit Rührvorrichtung aufweist, der stromauf mit einer Substratdosiervorrichtung und mit einer Enzymdosiervorrichtung verbunden ist und stromab mit mindestens einem Hydrolyserohr verbunden ist, wobei das Hydrolyserohr mit statischen Mischerelementen ausgerüstet ist und relativ große Abmessungen besitzt und der Behälter relativ kleine Abmessungen besitzt.

8. Vorrichtung nach Anspruch 7, bei der das Rohr vertikal angeordnet ist, wobei sein unteres Ende mit dem Behälter verbunden ist und sein oberes Ende in eine Austrittsleitung ausmündet.

9. Vorrichtung nach Anspruch 7, bei der die Substratdosiervorrichtung und die Enzymdosiervorrichtung jeweils einen mit dem Hydrolysebehälter verbundenen Versorgungsbehälter aufweisen.

10. Vorrichtung nach Anspruch 9, die außerdem eine Reagenzdosiervorrichtung aufweist, die einen Versorgungsbehälter besitzt, der mit dem Hydrolysebehälter über eine Zumesspumpe verbunden ist, die durch einen an den Hydrolysebehälter angeschlossenen pH-Messer gesteuert wird.

11. Vorrichtung nach Anspruch 10, die mehrere mit statischen Mischerelementen ausgerüstete Hydrolyserohre aufweist, die stromab des Behälters über Verbindungsleitungen in Reihe geschaltet sind, die stromauf mit der Enzymdosiervorrichtung und mit der Reagenzdosiervorrichtung verbunden sind.

## Claims

1. Process for the enzymatic hydrolysis of proteins, in which a protein substrate is subjected to a continuous hydrolysis with a proteolytic enzyme, a first step of relatively short enzymatic hydrolysis is performed in a stirred tank, and a second step of relatively long enzymatic hydrolysis is performed in a tube, the first step being performed for 10-60 minutes while adjusting the pH and the temperature to values that are favourable for the activity of the enzyme, the second step being performed for 1-8 hours while adjusting the temperature to a value greater than or equal to the temperature of the first step, and the second hydrolysis step being performed in a tube equipped with static mixing elements.

2. Process according to Claim 1, in which the said substrate is a protein-rich food raw material, especially flours or meals of oil-yielding seeds or cakes, food-grade yeasts or bacteria, minced animal or fish flesh, or milks or milk derivatives, in the form of particles in aqueous suspension or an aqueous suspension.

3. Process according to Claim 1, in which the said protein substrate is a whey substrate containing whey proteins, especially a mild whey from cheese production or an acidic whey from casein production, in unmodified form or in demineralized or lactose-freed, liquid or reconstituted form.

4. Process according to Claim 1, in which the proteolytic enzyme is chosen from the group formed by trypsin, chymotrypsin, pancreatin, bacterial proteases and fungal proteases, and mixtures thereof.

5. Process according to Claim 1, in which the proteolytic enzyme and the substrate are mixed together in a proportion of an amount of enzyme having an activity of 0.1-12 AU per 100 g of substrate dry matter.

6. Process according to Claim 1, in which the second step is performed by adjusting the temperature to a value equal to or 0-10°C above the temperature of the first step.

7. Apparatus for carrying out the process according to Claim 1, comprising a hydrolysis tank equipped with a stirrer, connected upstream to a substrate metering device and to an enzyme metering device, and connected downstream to at least one hydrolysis tube, the said hydrolysis tube being equipped with static mixing elements and being of relatively large size, and the tank being of relatively small size.

8. Apparatus according to Claim 7, in which the said tube is arranged vertically, its bottom end being connected to the said tank and its top end opening into an outlet pipe.

9. Apparatus according to Claim 7, in which the said substrate and enzyme metering devices each include a feed vessel connected to the said hydrolysis tank.

10. Apparatus according to Claim 9, also comprising a reagent metering device including a feed vessel connected to the hydrolysis tank via a positive-displacement pump controlled by a pH meter connected to the tank.

11. Apparatus according to Claim 10, comprising several hydrolysis tubes equipped with static mixing elements, connected in series downstream of the tank via connecting pipes connected upstream to the enzyme metering device and to the reagent metering device.
